# EUROPEAN PATENT APPLICATION

(11) **EP 1 526 180 A1**
(43) Date of publication of application: **27.04.2005**
(21) Application number: 03398007.9
(22) Date of filing: 17.10.2003
(51) Int. Cl.: C12N 15/52, C12N 9/10, C12N 9/16, C12N 5/10

(54) **Bifunctional gene for mannosylglycerate synthesis**

(71) Applicant: Stab Vida, Lda. Escola Superior de Biotecnologia/Univ. Catulica Portuguesa, 4200-072 Porto (PT)
(72) Inventor: Simoes Da Costa, Milton, 3000 Coimbra (PT); Miguel Da Silva Empadinhas, Nuno, 3000 Coimbra (PT); Da Costa Albuquerque Pinto, Luciana, 3000 Coimbra (PT); Santos, Helena, 2781-91 Oeiras (PT)

(57) **Abstract**

Mannosylglycerate (MG) is a mannose derivative compound that has been found in many thermophilic and hyperthermophilic microorganisms, where it accumulates concomitantly with salt stress conditions. This solute has been recently demonstrated as an excellent stabilizer of enzymes against thermal stress *in vitro.* To date, MG has been only detected in thermophilic or hyperthermophilic prokaryotes but not in mesophilic prokaryotes. The genes responsible for the synthesis of the compound only recently began to be characterized. We identified in the genome sequence from the mesophilic bacterium *Dehalococcoides ethenogenes* a bifunctional mannosyl-3-phosphoglycerate synthase (*mpgs*) and mannosyl-3-phosphoglycerate phosphatase (*mpgp*) gene. Cloning and transformation of this gene into *Saccharomyces cerevisiae* provided the organism with the ability to synthesize MG, which is suitable for industrial production of MG.

## Description

### TECHNICAL FIELD

The present invention relates to processes for producing mannosylglycerate by transformed organisms. It also relates to a bifunctional mannosylglycerate synthase protein or homologues, a mannosylglycerate synthase gene or homologues, recombinant plasmids carrying the said gene and transformed organisms with said recombinant plasmids.

The ultimate objective of this invention is to express a new bifunctional enzyme in an heterologous system in order to produce high amounts of manosylglicerate, an hypersolute which properties have been demonstrated to be industrially applicable.

### STATE OF THE ART

Mannosylglycerate, a mannose-derivative compatible solute accumulates to high levels, primarily in response to osmotic stress in many prokaryotic thermophilic and hyperthermophilic organisms [1]. Mannosylglycerate was originally identified in red algae of the family *Ceramiales* [2], but had never been identified in mesophilic prokaryotes. The organisms that accumulate MG range from the slightly halophilic euryarchaeotal archaea of the genera *Pyrococcus, Thermococcus* and *Archaeoglobus* to the crenarchaeote *Aeropyrum pernix* [3, *4*]. Among thermophilic bacteria MG has been identified in *Thermus thermophilus* and *Rhodothermus marinus* [5]. The role of MG as a compatible solute has been confirmed in these organisms as it accumulates concomitantly with the increase in salt concentration in the medium.

A fundamental protective role *in vivo* has been ascribed to MG from the observation of the invariable association of this solute with high temperatures, occurring exclusively in (hyper)thermophilic microorganisms. The effect on biological structures has been demonstrated in *vitro* and MG was proven to be one of the best protein stabilizers against thermal denaturation [6-8]. Unfortunately, the utilization of MG in stabilization studies of biological structures such as enzymes, DNA and whole cells against heat, osmotic stress and desiccation, has been greatly restricted because its production to date has been inefficient and expensive. The culture media for growing natural MG-producing microorganisms are quite complex cost-limiting and the conditions of temperature and salt required by such organisms are highly corrosive to industrial fermentation equipment. Moreover, natural producers are not suited for industrial production, as they do not grow to high cell densities. In conclusion, it would be desirable that the large-scale production of MG for biotechnological studies and applications would be developed under mesophilic conditions and by simple and low cost methodologies.

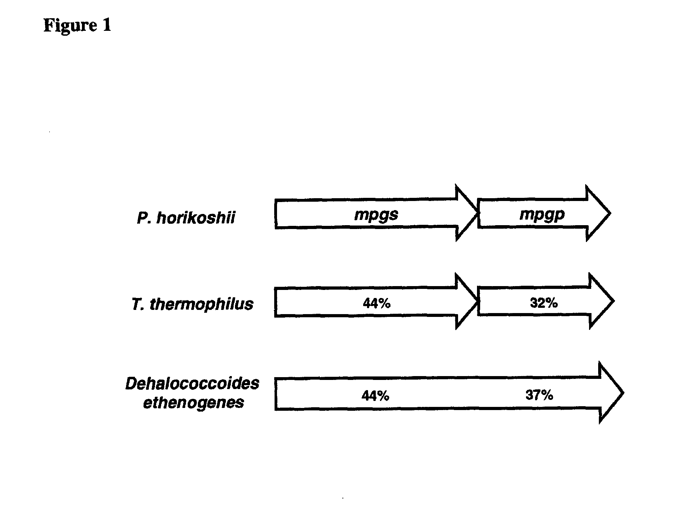

The pathways for the synthesis of compatible solutes in thermophilic and hyperthermophilic organisms have only recently began to be examined. The pathway for biosynthesis of MG, recently described in *Pyrococcus horikoshii* and *Thermus thermophilus,* consists on the condensation of GDP-mannose and D-3-phosphoglycerate into an intermediate phosphorylated compound, mannosyl-3-phosphoglycerate (MPG), which is then converted into MG. The enzymes responsible for the process, designated mannosyl-3-phosphoglycerate synthase (MPGS) and mannosyl-3-phosphoglycerate phosphatase (MPGP), are encoded by two consecutive genes in (hyper)thermophilic archaea and bacteria [9,10]. Surprisingly, as observed from the analysis of the genome sequence of the organism at the Institute for Genomic Research (TIGR), those coding sequences are also present in the mesophilic bacterium *Dehalococcoides ethenogenes,* but as a fusion of both genes. This open reading frame codes for a 78 kDa enzyme, containing two functional domains linked together by a short peptide (Fig 1), which is capable of catalyzing the two final steps in MG biosynthesis.

The inventors of this patent focused in finding a mesophilic producer of MG and enzymes which would work at moderate temperatures, to overproduce mannosylglycerate. *Dehalococcoides ethenogenes* has an optimum growth temperature of arround 30°C [11], and the referred gene appears as a valuable tool for those purposes..

*D. ethenogenes* is a strictly anaerobic organism, very difficult to cultivate due to its extremely complex nutritional requirements. The functional assignment of the gene as bifunctional mannosylglycerate synthase/phosphatase was accomplished by the combination of sequence analysis, in *vitro* data derived from the biochemical characterization of the enzyme expressed in *Escherichia coli,* as well as *in vivo* data resulting from the expression of the gene in a living organism, *Saccharomyces cerevisiae*. Limitations in enzyme supply from *D. ethenogenes* were overcome by the expression of the gene and overproduction of the enzyme in *E. coli*. The recombinant *E. coli* strain produced high amounts of soluble enzyme but did not lead to synthesis of MG *in vivo*. The recombinant *E. coli* strain produced high amounts of soluble enzyme but did not lead to synthesis of MG *in vivo*. Thus, the inventors pursued a second strategy based on enzyme overproduction in an alternative organism. *Saccharomyces cerevisiae* was the organism of choice since it was genetically easy to manipulate and had been a valuable biotechnological tool for the production of important compounds, as recently demonstrated, for example, with the complete biosynthesis of hydrocortisone [12].

The bifunctional gene was cloned into a *S. cerevisiae* vector and placed directly under the control of a strong constitutive promoter. Transformation of yeast cells with the plasmid containing the referred gene provided the organism with the capacity to synthesize and accumulate MG to a significant extent, leading to the current invention.

### DETAILED DESCRIPTION OF THE INVENTION

The process leading to the invention will better be portrayed through the use of examples:

### EXAMPLE 1

### Identification and isolation of a gene coding for a mesophilic and bifunctional mannosylglycerate synthase.

Analysis of the genome sequence (TIGR) of the bacterium *Dehalococcoides ethenogenes* lead to the identification of a sequence enclosing two functionally different domains, a mannosyl-3-phosphoglycerate synthase and a mannosyl-3-phosphoglycerate phosphatase. Searches and alignments were performed at http://www.ncbi.nlm.nih.gov with the gene with nucleotide sequence SEQ. ID N° 1, coding for a polypeptide with a predicted sequence shown in SEQ. ID N° 2. The corresponding protein sequence was different from its counterparts from (hyper)thermophilic bacteria where mannosyl-3-phosphoglycerate synthase and a mannosyl-3-phosphoglycerate phosphatase activities are processed by two independent proteins encoded by two independent genes. The amplification of the gene was performed by PCR with primers (SEQS. ID N° 3 AND 4). *EcoRI* and *HindIII* restriction sites (underlined) were added to the 5' primer terminals, respectively, and chromosomal DNA of the organism was used as template.

### EXAMPLE 2

### Cloning and overexpression of the bifunctional mannosylglycerate synthase gene in E. coli and synthesis of MG in vitro.

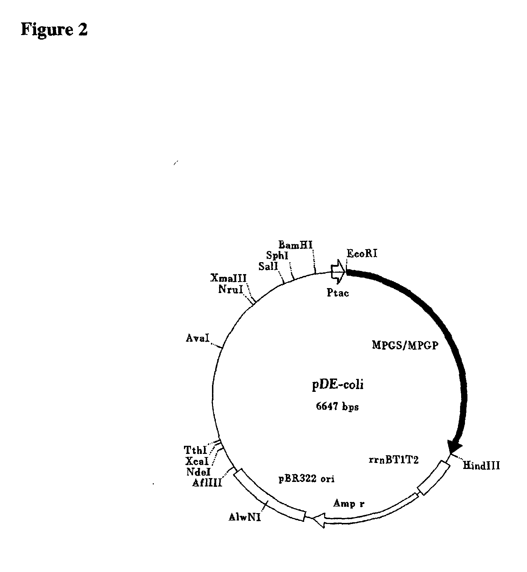

The amplification product obtained in example 1 was digested and ligated into the corresponding sites of the IPTG inducible *E. coli* expression vector pKK223-3 (Amersham-Pharmacia Biotech),yielding pDE-coli. The ligation mixture was transformed into *E. coli* XL1-blue competent cells and transformants were screened for the correct insertion of the gene by restriction analysis. Plasmids isolated from positive clones were sequenced to confirm the identity and position of the inserts (Fig. 2). These clones were cultivated and expression was induced with 1 mM IPTG for 12 h to achieve high gene expression levels. After disruption of the cells, protein-containing supernatant was analyzed by SDS-PAGE to evaluate the level of protein expression. *E. coli* cells exhibited an expression band of the expected size corresponding to the bifunctional mannosylglycerate synthase, not seen in the control extracts without the gene.

Cell extracts were incubated at 30°C for 30 min in 50 mM Tris-HCl buffer, pH 7.5, with the precursors for MG synthesis, GDP-mannose and 3-phosphoglycerate, in the presence of 10 mM MgCl₂. The reactions were spotted onto a TLC silica plate and developed with a solvent system composed of chloroform/methanol/acetic acid/water (8:4:2:1), heat-dried and stained with a-naphtol-sulfuric acid solution followed by charring at 120°C. The spots corresponding to MG were visible after 5 min. MG identity was confirmed by proton NMR.

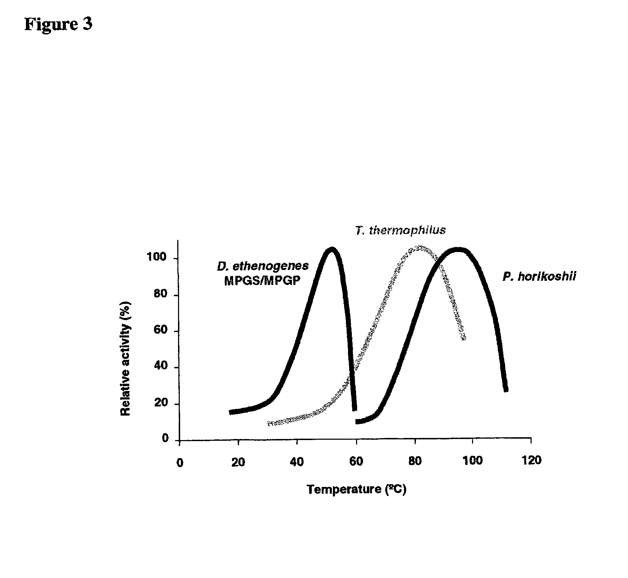

Overexpression of the bifunctional mannosylglycerate synthase in *E. coli* produced an enzyme capable of synthesis of *MG in vitro.* This enzyme was purified to homogeneity from cell extracts by chromatographic procedures and its properties were extensively studied. GDP-mannose and 3-phosphoglycerate were the exclusive substrates used by the enzyme to synthesize MG. The enzyme was found to be strictly dependent on cobalt ions to reach maximal activity and the optimal pH was around 7.0. Finally, the temperature profile of the enzyme was characteristic of a mesophilic enzyme. Its maximal activity was reached between 45 and 50°C, decreasing abruptly to near zero at 60°C (Fig 3). One activity unit (U) was defined as the amount of enzyme that converts 3 µmol of each substrate per minute, and per mg of enzyme, into mannosylglycerate. The inventors have thus provided a means of fully converting GDP-mannose and 3-phosphoglycerate into MG *in vitro* in one step. The optimal conditions for the reaction are: Buffer at pH 7.0, 2 mM CoCl₂, at 50°C.

### EXAMPLE 3

### Heterologous production of MG in Saccharomyces cerevisiae.

The amplification product obtained in example 1 with nucleotide sequence SEQ. ID N° 1, was re-amplified with primers SEQ. N° 5 and SEQ. N° 6 designed to include *HindIII* and *NruI* restriction sites (underlined). The obtained PCR fragment was digested with the corresponding restriction enzymes and cloned in the shuttle vector *E. coli*/*S*. *cerevisiae* pRS425, obtaining plasmid pDE. After transformation and selection of *E. coli* transformants, plasmids were then isolated.

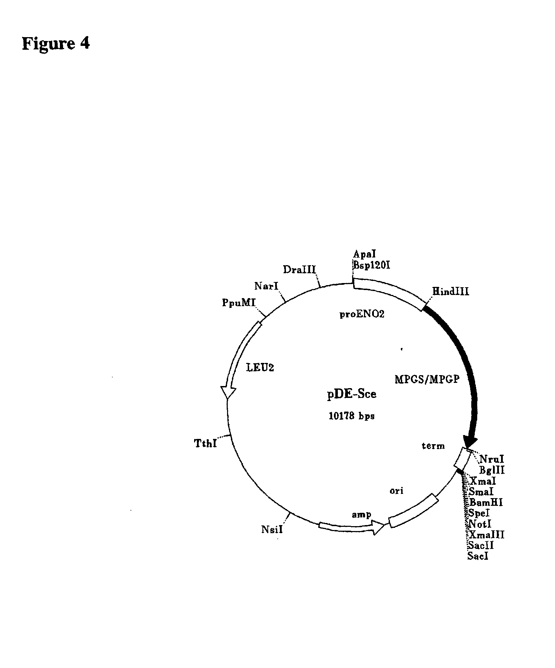

*Saccharomyces cerevisiae* enolase2 gene (ENO2) is controlled by a constitutive promoter that is highly efficient (SEQ. N° 7). The promoter sequence was amplified by PCR from *S. cerevisiae* chromosomal DNA with forward and reversed primers designed (SEQs. N° 8 and 9) to include the *ApaI* and *HindIII* sites respectively (underlined). Amplified ENO2 promoter DNA was digested and ligated into the corresponding sites of the previous construct, pDE, directly upstream from the fusion mannosylglycerate synthase gene (Fig 4), yielding plasmid pDE-Sce. A *Candida* sp. transcription termination sequence (SEQ. N° 10) had been previously inserted into the vector and was located immediately downstream the bifunctional mannosylglycerate synthase coding sequence. The selection procedure was the same as described above. The transformation of *S*. *cerevisiae* CEN-PK2 cells was performed by the lithium acetate method.

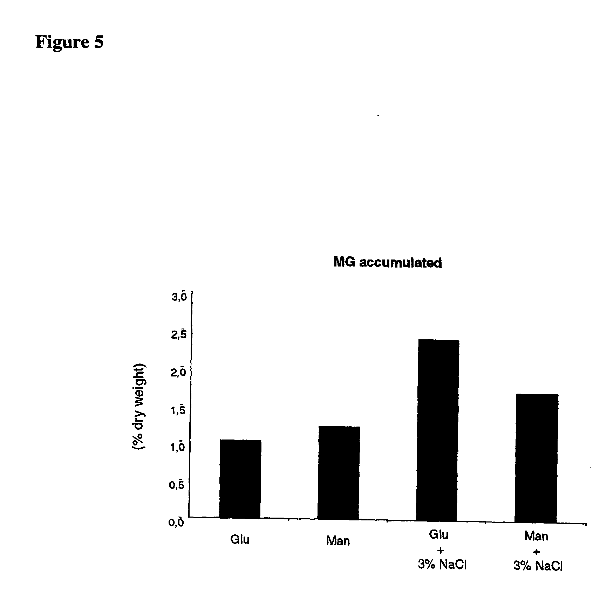

Cells were plated on minimal medium containing 2.0% glucose as the carbon source, 0.67% yeast nitrogen base without amino acids, and the auxotrophic requirements of the strain. Leucine was the exception since it used for selection of transformants and the plasmid contains a gene for the biosynthesis of this amino acid. Transformant colonies were used to inoculate liquid medium with the composition described above. At mid-exponential phase of growth (OD₆₀₀=2.0), the culture was cooled on ice and the suspension was centrifuged at 5000 rpm for 5 min. Cell biomass was ressuspended in PBS buffer to wash the cells from residual culture medium. The centrifugation step was repeated and cell biomass ressuspended in an exact amount of double distilled water. Organic solutes were ethanol extracted by the protocol described in patent No. EP0816509. Extracts were lyophilized, analyzed by ¹H-NMR and MG production was quantified as being 1.2% of cell dry-weight. The clones bearing the recombinant plasmid were cultivated in a medium with the composition described above except for the NaCl which was 3.0% (w/v). The culture conditions were the same, cells were collected by centrifugation as described above and organic solutes extracted by ethanol as described before. Analysis of extracts from *S. cerevisiae* cells growing in the presence of 3.0% NaCl and quantification of MG accumulated by ¹H-NMR, resulted in a 2 fold increase in the production of the solute (Fig 5).

### DISCLOSURE OF THE INVENTION

The present invention provides a gene coding for a novel bifunctional mannosylglycerate synthase. The preliminary sequence of the gene was obtained from the *Dehalococcoides ethenogenes* genome project at "The Institute for Genomic Research" through the website at http://www.tigr.org).

The present invention provides a bifunctional mannosylglycerate synthase protein able to synthesize mannosylglycerate *in vitro* in one step from GDP-mannose and 3-phosphoglycerate, at temperatures considerably lower than the growth temperatures of the natural producers described to date.

The present invention provides a recombinant plasmid pDE-coli in which a 2085 base pairs DNA fragment corresponding to the full-length gene encoding bifunctional mannosylglycerate synthase has been inserted behind the strong *tac* promoter into the *E. coli* expression vector pKK223-3 (Amersham Biosciences).

The present invention provides a transformed *E. coli* with a recombinant plasmid containing the bifunctional mannosylglycerate synthase gene allowing for high-level production of a bifunctional mannosylglycerate synthase protein with mesophilic character.

The present invention provides a recombinant plasmid pDE-Sce in which a 2085 base pairs PCR fragment, corresponding to the full length bifunctional mannosylglycerate synthase gene has been inserted into the yeast epissomal vector pRS425 behind a strong constitutive enolase (*ENO2*) promoter. This allows for high-level expression of the bifunctional mannosylglycerate synthase gene in *Saccharomyces cerevisiae*.

The present invention provides a procedure for synthesizing MG by incubating the bifunctional mannosylglycerate synthase protein with GDP-mannose and 3-phosphoglycerate in a buffered solution at a specific pH, containing a divalent cation as, for example, cobalt.

The present invention provides a process for producing mannosylglycerate by organisms from culture media containing a carbon source like glucose or mannose, a nitrogen base, and a small number of amino acid requirements, at a low temperature. *S. cerevisiae* CEN-PK2 transformed with plasmid pDE-Sce are grown to late-log phase to obtain a high amount of cell biomass, cells are centrifuged and mannosylglycerate extracted. Alternatively, S. *cerevisiae* cells can be cultivated in medium with the same composition described above to which 3.0% NaCl (w/v) is added. This way, the yield of MG accumulated is increased, comparing to cells cultivated in medium without salt.

### BRIEF DESCRIPTION OF THE FIGURES

**Figure 1** shows the degree of similarity between the translated sequence of the fusion gene from *Dehalococcoides ethenogenes* and the individual amino acid sequences coding for mannosyl-3-phosphoglycerate synthase and for mannosyl-3-phosphoglycerate phosphatase from *Pyrococcus furiosus* and from *Thermus thermophilus.*
**Figure 2** represents a plasmid able to replicate in *E. coli,* and was constructed by inserting the bifunctional mannosylglycerate synthase gene directly downstream the strong *tac* promoter, which upon IPTG induction enables high level expression of the gene under its control.
**Figure 3** ilustrates the temperature profile of the bifunctional mannosylglycerate synthase from *Dehalococcoides ethenogenes* produced and purified from *E. coli* cells, and the temperature profiles from the mannosyl-3-phosphoglycerate synthases from *Pyrococcus horikoshii* and *Thermus thermophilus,* based on the data of Empadinhas, N. *et al., J. Biol. Chem. 276,* 43580-43588 (2001), and of Empadinhas, N. *et al. Appl. Environ. Microbiol. 69*, 3272-3279 (2003).
**Figure 4** represents a shuttle plasmid able to replicate in *E. coli* and *Saccharomyces cerevisiae*, and was constructed by inserting a strong constitutive ENO2 promoter on the pRS425 vector, and by cloning the bifunctional mannosylglycerate synthase gene directly downstream this promoter sequence. The insertion of the gene places it directly in front of a yeast terminator sequence that has been previously inserted into the vector.
**Figure 5** shows the amount of mannosylglycerate produced and accumulated by *S. cerevisiae* cells, expressed as percentage of cell dry weight, in media containing a single carbon source and the appropriate auxotrophic requirements, in the absence or presence of NaCl (w/v).

### REFERENCES

[1] Santos, H., and M. S. da Costa. 2002. Compatible solutes of organisms that live in hot saline environments. Environ Microbiol. **4**:501-509.
[2] Kremer, B. P., and R. Vogl. 1975. Zur chemotaxonomichen bedung des (14C)-markierungsmusters bei Rhodophyceen. Phytochemistry **14**:1309-1314.
[3] Martins, L. O., and H. Santos. 1995. Accumulation of mannosylglycerate and di-myoinositol-phosphate by *Pyrococcus furiosus* in response to salinity and temperature Appl. Environ. Microbiol. **61**:3299-3303.
[4] Lamosa, P., L. O. Martins, M. S. da Costa, and H. Santos. 1998. Effects of temperature, salinity, and medium composition on compatible solute accumulation by *Thermococcus* spp. Appl. Environ. Microbiol. **64**:3591-3598.
[5] Nunes, O. C., C. M. Manaia, M. S. da Costa, and H. Santos. 1995. Compatible solutes in the thermophilic bacteria *Rhodothermus marinus* and "*Thermus thermophilus*". Appl. Environ. Microbiol. **61**:2351-2357.
[6] Ramos, A., N. D. H. Raven, R. J. Sharp, S. Bartolucci, M. Rossi, R. Cannio, J. Lebbink, J. Van der Oost, W. M. De Vos, and H. Santos. 1997. Stabilization of enzymes against thermal stress and freeze-drying by mannosylglycerate. Appl. Environ. MicrobioL **63**:4020-4025.
[7] Borges, N., A. Ramos, N. D. Raven, R. J. Sharp, and H. Santos. 2002. Comparative study of the thermostabilizing properties of mannosylglycerate and other compatible solutes on model enzymes. Extremophiles **6**:209-216.
[8] Santos, H., A. Ramos, and M. S. da Costa. 1998. Thermostabilization, osmoprotection, and protection against desiccation of enzymes, cell components and cells by mannosylglycerate. European Patent no. EP0816509.
[9] Empadinhas, N., J. D. Marugg, N. Borges, H. Santos, and M. S. da Costa. 2001. Pathway for the synthesis of mannosylglycerate in the hyperthermophilic archaeon *Pyrococcus horikoshii.* Biochemical and genetic characterization of key enzymes. J. Biol. Chem. **276**:43580-43588.
[10] Empadinhas N, L. Albuquerque, A. Henne, H. Santos and M. S. da Costa. 2003. The bacterium *Thermus thermophilus,* like hyperthermophilic archaea, uses a two-step pathway for the synthesis of mannosylglycerate. AppL Environ. Microbiol. **69**:3272-3279.
[11] Maymo-Gatell, X., Y. Chien, J. M. Gossett, and S. H. Zinder. 1997. Isolation of a bacterium that reductively dechlorinates tetrachloroethene to ethene. Science **276**:1568-1571.
[12] Szczebara FM, C. Chandelier, C. Villeret, A. Masurel, S. Bourot, C. Duport, S. Blanchard, A. Groisillier, E. Testet, P. Costaglioli, G. Cauet, E. Degryse, D. Balbuena, J. Winter, T. Achstetter, R Spagnoli, D. Pompon, and B. Dumas. 2003. Total biosynthesis of hydrocortisone from a simple carbon source in yeast. Nat. Biotechnol. **21**:143-149.

SEQUENCE LISTING

<110> STAB VIDA, Ltd.
<120> Identification of a bifunctional gene for manosylglicerate synthesis and
development of a high-scale-production heterologous system based on Sacharomyces
cerevisiae
<130> 01-00001
<140> EP03/03398007.9
<141> 2003-10-24
<160> 11
<210> 1
<211> 2085
<212> DNA
<213> Dehalococcoides ethenogenes
<220>
<221> CDS
<222> (1) ... (2082)
<300>
<309>
<400> 1
atg cgc att gaa agc ctg cgt ccc gcc gaa cac ctg ggg aat gta acc 48
Met Arg Ile Glu Ser Leu Arg Pro Ala Glu His Leu Gly Asn Val Thr
1               5                   10                  15
ata cac ggc gta agg cgt ttt tta gaa ctt gat tcc ggg gct ccc agc 96
Ile His Gly Val Arg Arg Phe Leu Glu Leu Asp Ser Gly Ala Pro Ser
            20                  25                  30
ctg cct cac ctg cag gaa agc cgt gag gtg gag atg gta gac caa cag 144
Leu Pro His Leu Gln Glu Ser Arg Glu Val Glu Met Val Asp Gln Gln
        35                  40                  45
gcc att tca gat gtt gaa aag aaa atg gcc att atc ctg ccc ata aaa 192
Ala Ile Ser Asp Val Glu Lys Lys Met Ala Ile Ile Leu Pro Ile Lys
    50                  55                  60
gac gaa gac ctt aaa gtt ttt gag ggt gtg ctg agc ggc ata ccc cat 240
Asp Glu Asp Leu Lys Val Phe Glu Gly Val Leu Ser Gly Ile Pro His
65                  70                  75                  80
gac tgt ttg atg ata gtt atc agc aac agc tcc aaa cag gaa gtg gat 288
Asp Cys Leu Met Ile Val Ile Ser Asn Ser Ser Lys Gln Glu Val Asp
                85                  90                  95
aac ttt aaa aac gaa aag gat att gtc agc cgc ttt tgc cgt ata acc 336
Asn Phe Lys Asn Glu Lys Asp Ile Val Ser Arg Phe Cys Arg Ile Thr
            100                 105                 110
cac cgc cag gcg ata gtt gtc cac cag aag aac ccc gaa ctg gca ggt 384
His Arg Gln Ala Ile Val Val His Gln Lys Asn Pro Glu Leu Ala Gly
        115                 120                 125
gcc att gca gat gcc ggc tac ccc gaa ctg ctg ggt aaa gac ggg ctt 432
Ala Ile Ala Asp Ala Gly Tyr Pro Glu Leu Leu Gly Lys Asp Gly Leu
    130                 135                 140
atc cgc agc ggc aaa gcc gag ggc atg ata ctg ggg att atc ctg act 480
Ile Arg Ser Gly Lys Ala Glu Gly Met Ile Leu Gly Ile Ile Leu Thr
145                 150                 155                 160
atg ttc agc ggg cgg gaa tac gtg ggt ttt ata gat acc gac aac tat 528
Met Phe Ser Gly Arg Glu Tyr Val Gly Phe Ile Asp Thr Asp Asn Tyr
                165                 170                 175
ata ccg gga gcg gtc tgg gaa tac gcc aaa cat ttt gct aca ggt ttt 576
Ile Pro Gly Ala Val Trp Glu Tyr Ala Lys His Phe Ala Thr Gly Phe

            180                 185                 190
aat ctg gct cag tca ccc tat tcc atg gtg cgg gtg ctc tgg aag tac 624
Asn Leu Ala Gln Ser Pro Tyr Ser Met Val Arg Val Leu Trp Lys Tyr
        195                 200                 205
aag ccc aag ctg gtg gga gac ctg tat ttc aaa cgc tgg ggc agg gtt 672
Lys Pro Lys Leu Val Gly Asp Leu Tyr Phe Lys Arg Trp Gly Arg Val
    210                 215                 220
tcc gag gtt acc aat aaa cac ctt aat cac ctg ata tcc agc aag ggc 720
Ser Glu Val Thr Asn Lys His Leu Asn His Leu Ile Ser Ser Lys Gly
225                 230                 235                 240
aag ttt gaa acc gag att atc aaa acc ggc aat gcg ggt gag cac gcc 768
Lys Phe Glu Thr Glu Ile Ile Lys Thr Gly Asn Ala Gly Glu His Ala
                245                 250                 255
atg agc ata gag ctg gct aaa cgc ctg act tac ggc acc ggc tat gcg 816
Met Ser Ile Glu Leu Ala Lys Arg Leu Thr Tyr Gly Thr Gly Tyr Ala
            260                 265                 270
gtg gaa acc aaa gag att atg tcc ata ctg gaa cag ttc agc ggg ata 864
Val Glu Thr Lys Glu Ile Met Ser Ile Leu Glu Gln Phe Ser Gly Ile
        275                 280                 285
ctg cct ata gag gac agg gaa gtg gcg gaa gaa ggg gtg gag ata ctc 912
Leu Pro Ile Glu Asp Arg Glu Val Ala Glu Glu Gly Val Glu Ile Leu
    290                 295                 300
caa acc gaa act atc aac ccc cac ctc cac gag gat aaa ggc ggt gac 960
Gln Thr Glu Thr Ile Asn Pro His Leu His Glu Asp Lys Gly Gly Asp
305                 310                 315                 320
cac ctg ata cag gat atg ctt ttg ccc agc ctg gca gtt atc tat cac 1008
His Leu Ile Gln Asp Met Leu Leu Pro Ser Leu Ala Val Ile Tyr His
                325                 330                 335
agc ccg ctg gcg gat gaa agc gtg aaa aaa aag att gaa aac cag ctg 1056
Ser Pro Leu Ala Asp Glu Ser Val Lys Lys Lys Ile Glu Asn Gln Leu
            340                 345                 350
gca gga att gag ggg ctg gaa aac aac cct gag ata ccc cag gta aag 1104
Ala Gly Ile Glu Gly Leu Glu Asn Asn Pro Glu Ile Pro Gln Val Lys
        355                 360                 365
ctg att ccc ccg ccc cag aag atg gac ctg gcg aag ttt tca gcc gtt 1152
Leu Ile Pro Pro Pro Gln Lys Met Asp Leu Ala Lys Phe Ser Ala Val
    370                 375                 380
att gaa aag tat ctg ccc cag atg gtt ctg cct gac gga gag ctg att 1200
Ile Glu Lys Tyr Leu Pro Gln Met Val Leu Pro Asp Gly Glu Leu Ile
385                 390                 395                 400
tca cgg ata gcc cgc ccc tcc cgg ctg ccg tct tcg gga cag ttt aaa 1248
Ser Arg Ile Ala Arg Pro Ser Arg Leu Pro Ser Ser Gly Gln Phe Lys
                405                 410                 415
aag gtt atc tat act gat ttg gac ggc act ttg ctt agc ccc ctg act 1296
Lys Val Ile Tyr Thr Asp Leu Asp Gly Thr Leu Leu Ser Pro Leu Thr
            420                 425                 430
tat tca tat tca acc gca ctg gat gct ttg cgg ctg ctc aaa gac aaa 1344
Tyr Ser Tyr Ser Thr Ala Leu Asp Ala Leu Arg Leu Leu Lys Asp Lys
        435                 440                 445
gag ctg cca ctg gta ttc tgc tct gcc aag act atg ggc gag caa gac 1392
Glu Leu Pro Leu Val Phe Cys Ser Ala Lys Thr Met Gly Glu Gln Asp

450                 455                 460
ctg tac cgc aac gaa ctg ggt atc aaa gac ccc ttt ata acc gaa aac          1440
Leu Tyr Arg Asn Glu Leu Gly Ile Lys Asp Pro Phe Ile Thr Glu Asn
465                 470                 475                 480
ggg gga gct atc ttt atc ccc aaa gat tac ttc cgc ctg ccc ttt gcc          1488
Gly Gly Ala Ile Phe Ile Pro Lys Asp Tyr Phe Arg Leu Pro Phe Ala
                485                 490                 495
tat gac aga gta gcc gga aat tat ctg gtg att gag ctg ggt atg agc          1536
Tyr Asp Arg Val Ala Gly Asn Tyr Leu Val Ile Glu Leu Gly Met Ser
            500                 505                 510
tat aaa gat atc cgc cat att ctt aaa aaa gcc ctg gcc gag gcc tgt          1584
Tyr Lys Asp Ile Arg His Ile Leu Lys Lys Ala Leu Ala Glu Ala Cys
        515                 520                 525
acc gaa ata gaa aac tct gaa aga gcc ggc aat ata ttt att acc agc          1632
Thr Glu Ile Glu Asn Ser Glu Arg Ala Gly Asn Ile Phe Ile Thr Ser
    530                 535                 540
ttc ggg gat atg tct gtt gaa gac gtc agc cgc ctt act gac ctt aac          1680
Phe Gly Asp Met Ser Val Glu Asp Val Ser Arg Leu Thr Asp Leu Asn
545                 550                 555                 560
ctg aag cag gcc gag ctg gcc aaa cag cgt gaa tac agc gag acc gta          1728
Leu Lys Gln Ala Glu Leu Ala Lys Gln Arg Glu Tyr Ser Glu Thr Val
                565                 570                 575
cac ata gag ggc gat aaa cgc agt acc aat ata gtc ctg aat cat atc          1776
His Ile Glu Gly Asp Lys Arg Ser Thr Asn Ile Val Leu Asn His Ile
            580                 585                 590
cag caa aac ggg ctg gaa tac agt ttc ggg ggg cgt ttc tac gaa gtc          1824
Gln Gln Asn Gly Leu Glu Tyr Ser Phe Gly Gly Arg Phe Tyr Glu Val
        595                 600                 605
acc ggc ggc aat gac aag ggc aag gcc ata aag gta ctg aac gag ctg          1872
Thr Gly Gly Asn Asp Lys Gly Lys Ala Ile Lys Val Leu Asn Glu Leu
    610                 615                 620
ttc cgc ctg aat ttc ggc aat atc cac acc ttt ggt ttg ggt gac agc          1920
Phe Arg Leu Asn Phe Gly Asn Ile His Thr Phe Gly Leu Gly Asp Ser
625                 630                 635                 640
gaa aat gac tac tcc atg ctg gaa aca gtg gat tcc ccc ata ctg gtg          1968
Glu Asn Asp Tyr Ser Met Leu Glu Thr Val Asp Ser Pro Ile Leu Val
                645                 650                 655
caa cgc ccc ggc aac aag tgg cac aag atg cgt ttg cgt aac ccc agt          2016
Gln Arg Pro Gly Asn Lys Trp His Lys Met Arg Leu Arg Asn Pro Ser
            660                 665                 670
tat gtc aga ggg gta ggg ccg gag ggt ttc agc cgg gca gtt acc gat          2064
Tyr Val Arg Gly Val Gly Pro Glu Gly Phe Ser Arg Ala Val Thr Asp
        675                 680                 685
ata ata ctg ccc atg gaa taa                                              2085
Ile Ile Leu Pro Met Glu -
    690

<210> 2
<211> 694
<212> PRT
<213> Dehalococcoides ethenogenes
<400> 2

Met Arg Ile Glu Ser Leu Arg Pro Ala Glu His Leu Gly Asn Val Thr

1               5                   10                  15
Ile His Gly Val Arg Arg Phe Leu Glu Leu Asp Ser Gly Ala Pro Ser
            20                  25                  30
Leu Pro His Leu Gln Glu Ser Arg Glu Val Glu Met Val Asp Gln Gln
        35                  40                  45
Ala Ile Ser Asp Val Glu Lys Lys Met Ala Ile Ile Leu Pro Ile Lys
    50                  55                  60
Asp Glu Asp Leu Lys Val Phe Glu Gly Val Leu Ser Gly Ile Pro His
65                  70                  75                  80
Asp Cys Leu Met Ile Val Ile Ser Asn Ser Ser Lys Gln Glu Val Asp
                85                  90                  95
Asn Phe Lys Asn Glu Lys Asp Ile Val Ser Arg Phe Cys Arg Ile Thr
            100                 105                 110
His Arg Gln Ala Ile Val Val His Gln Lys Asn Pro Glu Leu Ala Gly
        115                 120                 125
Ala Ile Ala Asp Ala Gly Tyr Pro Glu Leu Leu Gly Lys Asp Gly Leu
    130                 135                 140
Ile Arg Ser Gly Lys Ala Glu Gly Met Ile Leu Gly Ile Ile Leu Thr
145                 150                 155                 160
Met Phe Ser Gly Arg Glu Tyr Val Gly Phe Ile Asp Thr Asp Asn Tyr
                165                 170                 175
Ile Pro Gly Ala Val Trp Glu Tyr Ala Lys His Phe Ala Thr Gly Phe
            180                 185                 190
Asn Leu Ala Gln Ser Pro Tyr Ser Met Val Arg Val Leu Trp Lys Tyr
        195                 200                 205
Lys Pro Lys Leu Val Gly Asp Leu Tyr Phe Lys Arg Trp Gly Arg Val
    210                 215                 220
Ser Glu Val Thr Asn Lys His Leu Asn His Leu Ile Ser Ser Lys Gly
225                 230                 235                 240
Lys Phe Glu Thr Glu Ile Ile Lys Thr Gly Asn Ala Gly Glu His Ala
                245                 250                 255
Met Ser Ile Glu Leu Ala Lys Arg Leu Thr Tyr Gly Thr Gly Tyr Ala
            260                 265                 270
Val Glu Thr Lys Glu Ile Met Ser Ile Leu Glu Gln Phe Ser Gly Ile
        275                 280                 285
Leu Pro Ile Glu Asp Arg Glu Val Ala Glu Glu Gly Val Glu Ile Leu
    290                 295                 300
Gln Thr Glu Thr Ile Asn Pro His Leu His Glu Asp Lys Gly Gly Asp
305                 310                 315                 320
His Leu Ile Gln Asp Met Leu Leu Pro Ser Leu Ala Val Ile Tyr His
                325                 330                 335
Ser Pro Leu Ala Asp Glu Ser Val Lys Lys Lys Ile Glu Asn Gln Leu
            340                 345                 350
Ala Gly Ile Glu Gly Leu Glu Asn Asn Pro Glu Ile Pro Gln Val Lys
        355                 360                 365
Leu Ile Pro Pro Pro Gln Lys Met Asp Leu Ala Lys Phe Ser Ala Val
    370                 375                 380
Ile Glu Lys Tyr Leu Pro Gln Met Val Leu Pro Asp Gly Glu Leu Ile
385                 390                 395                 400
Ser Arg Ile Ala Arg Pro Ser Arg Leu Pro Ser Ser Gly Gln Phe Lys
                405                 410                 415

Lys Val Ile Tyr Thr Asp Leu Asp Gly Thr Leu Leu Ser Pro Leu Thr
            420                 425                 430
Tyr Ser Tyr Ser Thr Ala Leu Asp Ala Leu Arg Leu Leu Lys Asp Lys
        435                 440                 445
Glu Leu Pro Leu Val Phe Cys Ser Ala Lys Thr Met Gly Glu Gln Asp
    450                 455                 460
Leu Tyr Arg Asn Glu Leu Gly Ile Lys Asp Pro Phe Ile Thr Glu Asn
465                 470                 475                 480
Gly Gly Ala Ile Phe Ile Pro Lys Asp Tyr Phe Arg Leu Pro Phe Ala
                485                 490                 495
Tyr Asp Arg Val Ala Gly Asn Tyr Leu Val Ile Glu Leu Gly Met Ser
            500                 505                 510
Tyr Lys Asp Ile Arg His Ile Leu Lys Lys Ala Leu Ala Glu Ala Cys
        515                 520                 525
Thr Glu Ile Glu Asn Ser Glu Arg Ala Gly Asn Ile Phe Ile Thr Ser
    530                 535                 540
Phe Gly Asp Met Ser Val Glu Asp Val Ser Arg Leu Thr Asp Leu Asn
545                 550                 555                 560
Leu Lys Gln Ala Glu Leu Ala Lys Gln Arg Glu Tyr Ser Glu Thr Val
                565                 570                 575
His Ile Glu Gly Asp Lys Arg Ser Thr Asn Ile Val Leu Asn His Ile
            580                 585                 590
Gln Gln Asn Gly Leu Glu Tyr Ser Phe Gly Gly Arg Phe Tyr Glu Val
        595                 600                 605
Thr Gly Gly Asn Asp Lys Gly Lys Ala Ile Lys Val Leu Asn Glu Leu
    610                 615                 620
Phe Arg Leu Asn Phe Gly Asn Ile His Thr Phe Gly Leu Gly Asp Ser
625                 630                 635                 640
Glu Asn Asp Tyr Ser Met Leu Glu Thr Val Asp Ser Pro Ile Leu Val
                645                 650                 655
Gln Arg Pro Gly Asn Lys Trp His Lys Met Arg Leu Arg Asn Pro Ser
            660                 665                 670
Tyr Val Arg Gly Val Gly Pro Glu Gly Phe Ser Arg Ala Val Thr Asp
        675                 680                 685
Ile Ile Leu Pro Met Glu
    690

<210> 3
<211> 32
<212> DNA
<213> Artificial Sequence
<220>
<400> 3

gcgaattcat gcgcattgaa agcctgcgtc cc 32

<210> 4
<211> 35
<212> DNA
<213> Artificial Sequence
<220>
<400> 4
gcgaagcttt tattccatgg gcagtattat atcgg 35

<210> 5
<211> 29
<212> DNA
<213> Artificial Sequence
<220>
<400> 5
gtcaagctta tgcgcattga aagcctgcg 29

<210> 6
<211> 33
<212> DNA
<213> Artificial Sequence
<220>
<400> 6
gtctcgcgat tattccatgg gcagtattat atc 33

<210> 7
<211> 32
<212> DNA
<213> Artificial Sequence
<220>
<400> 7
cggcccaatc ctactcttgc cgttgccatc caaaatgagc tagaaggtgg attaacaaat 60
ataatgacaa atcgttgctt gtctgacttg attccactac agttacaaat atttgacatt 120
gtatataagt tttgcaagtt catcaaatct atgagagcaa aattatgtca actggacccc 180
gtactatatg agaaacacaa aagcgggatg atgaaaacac taaacgaagg ctatcgtaca 240
aacaatggcg gtcaggaaga tgttggttac caagaagatg ccgccctgga attaattcag 300
aagctgattg aatacattag caacgcgtcc agcatttttc ggaagtgtct cataaacttt 360
actcaagagt taagtactga aaaattcgac ttttatgata gttcaagtgt cgacgctgcg 420
ggtatagaaa gggttcttta ctctatagta cctcctcgct cagcatctgc ttcttcccaa 480
agatgaacgc ggcgttatgt cactaacgac gtgcaccaac ttgcggaaag tggaatcccg 540
ttccaaaact ggcatccact aattgataca tctacacacc gcacgccttt tttctgaagc 600
ccactttcgt ggactttgcc atatgcaaaa ttcatgaagt gtgataccaa gtcagcatac 660
acctcactag ggtagtttct ttggttgtat tgatcatttg gttcatcgtg gttcattaat 720
tttttttctc cattgctttc tggctttgat cttactatca tttggatttt tgtcgaaggt 780
tgtagaattg tatgtgacaa gtggcaccaa gcatatataa aaaaaaaaag cattatcttc 840
ctaccagagt tgattgttaa aaacgtattt atagcaaacg caattgtaat taattcttat 900
tttgtatctt ttcttccctt gtctcaatct tttattttta ttttattttt cttttcttag 960
tttctttcat aacaccaagc aactaatact ataacataca ataataaagc tt 1012

<210> 8
<211> 32
<212> DNA
<213> Artificial Sequence
<400> 8
agctagggcc caatcctact cttgccgttg cc 32
<210> 9
<211> 40
<212> DNA
<213> Artificial Sequence
<400> 9
gatctaagct ttattattgt atgttatagt attagttgct 40

<210> 10
<211> 286
<212> DNA
<213> Artificial Sequence
<400> 10
tcgcgagcta gttgaatatt atgtaagatc tgttagagtt tttattttgt attcatttat 60

ttagtttatt ttcttattat atttgaaata taatatatat tttaaaaaaa aaatttacag 120
tgtagaattt ttggtagtcc ggtttgtttt gaaatcagtg gtggtattca atatttgatt 180
aaattttggt atgaatttgt gttgaaaaat aaaaaataag cgagaaattt gcgtggcata 240
ttatttgtaa tgttcgaata ttctctcgta caccaaaagg ctgcag 286

<210> 11
<400> 11
000

SEQUENCES

SEQ. ID N° 1

1 ATG CGC ATT GAA AGC CTG CGT CCC GCC GAA CAC CTG GGG AAT GTA ACC
49 ATA CAC GGC GTA AGG CGT TTT TTA GAA CTT GAT TCC GGG GCT CCC AGC
97 CTG CCT CAC CTG CAG GAA AGC CGT GAG GTG GAG ATG GTA GAC CAA CAG
145 GCC ATT TCA GAT GTT GAA AAG AAA ATG GCC ATT ATC CTG CCC ATA AAA
193 GAC GAA GAC CTT AAA GTT TTT GAG GGT GTG CTG AGC GGC ATA CCC CAT
241 GAC TGT TTG ATG ATA GTT ATC AGC AAC AGC TCC AAA CAG GAA GTG GAT
289 AAC TTT AAA AAC GAA AAG GAT ATT GTC AGC CGC TTT TGC CGT ATA ACC
337 CAC CGC CAG GCG ATA GTT GTC CAC CAG AAG AAC CCC GAA CTG GCA GGT
385 GCC ATT GCA GAT GCC GGC TAC CCC GAA CTG CTG GGT AAA GAC GGG CTT
433 ATC CGC AGC GGC AAA GCC GAG GGC ATG ATA CTG GGG ATT ATC CTG ACT
481 ATG TTC AGC GGG CGG GAA TAC GTG GGT TTT ATA GAT ACC GAC AAC TAT
529 ATA CCG GGA GCG GTC TGG GAA TAC GCC AAA CAT TTT GCT ACA GGT TTT
577 AAT CTG GCT CAG TCA CCC TAT TCC ATG GTG CGG GTG CTC TGG AAG TAC
625 AAG CCC AAG CTG GTG GGA GAC CTG TAT TTC AAA CGC TGG GGC AGG GTT
673 TCC GAG GTT ACC AAT AAA CAC CTT AAT CAC CTG ATA TCC AGC AAG GGC
721 AAG TTT GAA ACC GAG ATT ATC AAA ACC GGC AAT GCG GGT GAG CAC GCC
769 ATG AGC ATA GAG CTG GCT AAA CGC CTG ACT TAC GGC ACC GGC TAT GCG
817 GTG GAA ACC AAA GAG ATT ATG TCC ATA CTG GAA CAG TTC AGC GGG ATA
865 CTG CCT ATA GAG GAC AGG GAA GTG GCG GAA GAA GGG GTG GAG ATA CTC
913 CAA ACC GAA ACT ATC AAC CCC CAC CTC CAC GAG GAT AAA GGC GGT GAC
961 CAC CTG ATA CAG GAT ATG CTT TTG CCC AGC CTG GCA GTT ATC TAT CAC
1009 AGC CCG CTG GCG GAT GAA AGC GTG AAA AAA AAG ATT GAA AAC CAG CTG
1057 GCA GGA ATT GAG GGG CTG GAA AAC AAC CCT GAG ATA CCC CAG GTA AAG
1105 CTG ATT CCC CCG CCC CAG AAG ATG GAC CTG GCG AAG TTT TCA GCC GTT
1153 ATT GAA AAG TAT CTG CCC CAG ATG GTT CTG CCT GAC GGA GAG CTG ATT
1201 TCA CGG ATA GCC CGC CCC TCC CGG CTG CCG TCT TCG GGA CAG TTT AAA
1249 AAG GTT ATC TAT ACT GAT TTG GAC GGC ACT TTG CTT AGC CCC CTG ACT
1297 TAT TCA TAT TCA ACC GCA CTG GAT GCT TTG CGG CTG CTC AAA GAC AAA
1345 GAG CTG CCA CTG GTA TTC TGC TCT GCC AAG ACT ATG GGC GAG CAA GAC
1393 CTG TAC CGC AAC GAA CTG GGT ATC AAA GAC CCC TTT ATA ACC GAA AAC
1441 GGG GGA GCT ATC TTT ATC CCC AAA GAT TAC TTC CGC CTG CCC TTT GCC
1489 TAT GAC AGA GTA GCC GGA AAT TAT CTG GTG ATT GAG CTG GGT ATG AGC
1537 TAT AAA GAT ATC CGC CAT ATT CTT AAA AAA GCC CTG GCC GAG GCC TGT
1585 ACC GAA ATA GAA AAC TCT GAA AGA GCC GGC AAT ATA TTT ATT ACC AGC
1633 TTC GGG GAT ATG TCT GTT GAA GAC GTC AGC CGC CTT ACT GAC CTT AAC
1681 CTG AAG CAG GCC GAG CTG GCC AAA CAG CGT GAA TAC AGC GAG ACC GTA
1729 CAC ATA GAG GGC GAT AAA CGC AGT ACC AAT ATA GTC CTG AAT CAT ATC
1777 CAG CAA AAC GGG CTG GAA TAC AGT TTC GGG GGG CGT TTC TAC GAA GTC
1825 ACC GGC GGC AAT GAC AAG GGC AAG GCC ATA AAG GTA CTG AAC GAG CTG
1873 TTC CGC CTG AAT TTC GGC AAT ATC CAC ACC TTT GGT TTG GGT GAC AGC
1921 GAA AAT GAC TAC TCC ATG CTG GAA ACA GTG GAT TCC CCC ATA CTG GTG
1969 CAA CGC CCC GGC AAC AAG TGG CAC AAG ATG CGT TTG CGT AAC CCC AGT

2017  TAT GTC AGA GGG GTA GGG CCG GAG GGT TTC AGC CGG GCA GTT ACC GAT
2065  ATA ATA CTG CCC ATG GAA TAA

SEQ. ID N° 2
Met Arg Ile Glu Ser Leu Arg Pro Ala Glu His Leu Gly Asn Val Thr Ile His Gly Val
                5                   10                  15                  20
Arg Arg Phe Leu Glu Leu Asp Ser Gly Ala Pro Ser Leu Pro His Leu Gln Glu Ser Arg
                25                  30                  35                  40
Glu Val Glu Met Val Asp Gln Gln Ala Ile Ser Asp Val Glu Lys Lys Met Ala Ile Ile
                45                  50                  55                  60
Leu Pro Ile Lys Asp Glu Asp Leu Lys Val Phe Glu Gly Val Leu Ser Gly Ile Pro His
                65                  70                  75                  80
Asp Cys Leu Met Ile Val Ile Ser Asn Ser Ser Lys Gln Glu Val Asp Asn Phe Lys Asn
                85                  90                  95                  100
Glu Lys Asp Ile Val Ser Arg Phe Cys Arg Ile Thr His Arg Gln Ala Ile Val Val His
                105                 110                 115                 120
Gln Lys Asn Pro Glu Leu Ala Gly Ala Ile Ala Asp Ala Gly Tyr Pro Glu Leu Leu Gly
                125                 130                 135                 140
Lys Asp Gly Leu Ile Arg Ser Gly Lys Ala Glu Gly Met Ile Leu Gly Ile Ile Leu Thr
                145                 150                 155                 160
Met Phe Ser Gly Arg Glu Tyr Val Gly Phe Ile Asp Thr Asp Asn Tyr Ile Pro Gly Ala
                165                 170                 175                 180
Val Trp Glu Tyr Ala Lys His Phe Ala Thr Gly Phe Asn Leu Ala Gln Ser Pro Tyr Ser
                185                 190                 195                 200
Met Val Arg Val Leu Trp Lys Tyr Lys Pro Lys Leu Val Gly Asp Leu Tyr Phe Lys Arg
                205                 210                 215                 220
Trp Gly Arg Val Ser Glu Val Thr Asn Lys His Leu Asn His Leu Ile Ser Ser Lys Gly
                225                 230                 235                 240
Lys Phe Glu Thr Glu Ile Ile Lys Thr Gly Asn Ala Gly Glu His Ala Met Ser Ile Glu
                245                 250                 255                 260
Leu Ala Lys Arg Leu Thr Tyr Gly Thr Gly Tyr Ala Val Glu Thr Lys Glu Ile Met Ser
                265                 270                 275                 280
Ile Leu Glu Gln Phe Ser Gly Ile Leu Pro Ile Glu Asp Arg Glu Val Ala Glu Glu Gly
                285                 290                 295                 300
Val Glu Ile Leu Gln Thr Glu Thr Ile Asn Pro His Leu His Glu Asp Lys Gly Gly Asp
                305                 310                 315                 320
His Leu Ile Gln Asp Met Leu Leu Pro Ser Leu Ala Val Ile Tyr His Ser Pro Leu Ala
                325                 330                 335                 340
Asp Glu Ser Val Lys Lys Lys Ile Glu Asn Gln Leu Ala Gly Ile Glu Gly Leu Glu Asn
                345                 350                 355                 360
Asn Pro Glu Ile Pro Gln Val Lys Leu Ile Pro Pro Pro Gln Lys Met Asp Leu Ala Lys
                365                 370                 375                 380
Phe Ser Ala Val Ile Glu Lys Tyr Leu Pro Gln Met Val Leu Pro Asp Gly Glu Leu Ile
                385                 390                 395                 400
Ser Arg Ile Ala Arg Pro Ser Arg Leu Pro Ser Ser Gly Gln Phe Lys Lys Val Ile Tyr

                405                 410                 415                 420
Thr Asp Leu Asp Gly Thr Leu Leu Ser Pro Leu Thr Tyr Ser Tyr Ser Thr Ala Leu Asp
                425                 430                 435                 440
Ala Leu Arg Leu Leu Lys Asp Lys Glu Leu Pro Leu Val Phe Cys Ser Ala Lys Thr Met
                445                 450                 455                 460
Gly Glu Gln Asp Leu Tyr Arg Asn Glu Leu Gly Ile Lys Asp Pro Phe Ile Thr Glu Asn
                465                 470                 475                 480
Gly Gly Ala Ile Phe Ile Pro Lys Asp Tyr Phe Arg Leu Pro Phe Ala Tyr Asp Arg Val
                485                 490                 495                 500
Ala Gly Asn Tyr Leu Val Ile Glu Leu Gly Met Ser Tyr Lys Asp Ile Arg His Ile Leu
                505                 510                 515                 520
Lys Lys Ala Leu Ala Glu Ala Cys Thr Glu Ile Glu Asn Ser Glu Arg Ala Gly Asn Ile
                525                 530                 535                 540
Phe Ile Thr Ser Phe Gly Asp Met Ser Val Glu Asp Val Ser Arg Leu Thr Asp Leu Asn
                545                 550                 555                 560
Leu Lys Gln Ala Glu Leu Ala Lys Gln Arg Glu Tyr Ser Glu Thr Val His Ile Glu Gly
                565                 570                 575                 580
Asp Lys Arg Ser Thr Asn Ile Val Leu Asn His Ile Gln Gln Asn Gly Leu Glu Tyr Ser
                585                 590                 595                 600
Phe Gly Gly Arg Phe Tyr Glu Val Thr Gly Gly Asn Asp Lys Gly Lys Ala Ile Lys Val
                605                 610                 615                 620
Leu Asn Glu Leu Phe Arg Leu Asn Phe Gly Asn Ile His Thr Phe Gly Leu Gly Asp Ser
                625                 630                 635                 640
Glu Asn Asp Tyr Ser Met Leu Glu Thr Val Asp Ser Pro Ile Leu Val Gln Arg Pro Gly
                645                 650                 655                 660
Asn Lys Trp His Lys Met Arg Leu Arg Asn Pro Ser Tyr Val Arg Gly Val Gly Pro Glu
                665                 670                 675                 680
Gly Phe Ser Arg Ala Val Thr Asp Ile Ile Leu Pro Met Glu
                685                 690

SEQ. ID N° 3
5'-GCGGAATTCATGCGCATTGAAAGCCTGCGTCCC-3'

SEQ. ID N° 4
5'-GCGAAGCTTTTATTCCATGGGCAGTATTATATCGG-3'

SEQ. ID N° 5
5'-GTCAAGCTTATGCGCATTGAAAGCCTGCG-3'

SEQ. ID N° 6
5'-GTCTCGCGATTATTCCATGGGCAGTATTATATC-3'

SEQ. ID N° 7

GGGCCCAATCCTACTCTTGCCGTTGCCATCCAAAATGAGCTAGAAGGTGGATTAACAAATATAATGACAAATCGTTGCTTGTCTGACTT
GATTCCACTACAGTTACAAATATTTGACATTGTATATAAGTTTTGCAAGTTCATCAAATCTATGAGAGCAAAATTATGTCAACTGGACC
CCGTACTATATGAGAAACACAAAAGCGGGATGATGAAAACACTAAACGAAGGCTATCGTACAAACAATGGCGGTCAGGAAGATGTTGGT
TACCAAGAAGATGCCGCCCTGGAATTAATTCAGAAGCTGATTGAATACATTAGCAACGCGTCCAGCATTTTTCGGAAGTGTCTCATAAA
CTTTACTCAAGAGTTAAGTACTGAAAAATTCGACTTTTATGATAGTTCAAGTGTCGACGCTGCGGGTATAGAAAGGGTTCTTTACTCTA
TAGTACCTCCTCGCTCAGCATCTGCTTCTTCCCAAAGATGAACGCGGCGTTATGTCACTAACGACGTGCACCAACTTGCGGAAAGTGGA
ATCCCGTTCCAAAACTGGCATCCACTAATTGATACATCTACACACCGCACGCCTTTTTTCTGAAGCCCACTTTCGTGGACTTTGCCATA
TGCAAAATTCATGAAGTGTGATACCAAGTCAGCATACACCTCACTAGGGTAGTTTCTTTGGTTGTATTGATCATTTGGTTCATCGTGGT
TCATTAATTTTTTTTCTCCATTGCTTTCTGGCTTTGATCTTACTATCATTTGGATTTTTGTCGAAGGTTGTAGAATTGTATGTGACAAG
TGGCACCAAGCATATATAAAAAAAAAAAGCATTATCTTCCTACCAGAGTTGATTGTTAAAAACGTATTTATAGCAAACGCAATTGTAAT
TAATTCTTATTTTGTATCTTTTCTTCCCTTGTCTCAATCTTTTATTTTTATTTTATTTTTCTTTTCTTAGTTTCTTTCATAACACCAAG
CAACTAATACTATAACATACAATAATAAAGCTT

SEQ. ID N° 8

5'-AGCTAGGGCCCAATCCTACTCTTGCCGTTGCC-3'

SEQ. ID N° 9

5'-GATCTAAGCTTTATTATTGTATGTTATAGTATTAGTTGCT-3'

SEQ. ID N° 10

TCGCGAGCTAGTTGAATATTATGTAAGATCTGTTAGAGTTTTTATTTTGTATTCATTTATTTAGTTTATTTTCTTATTATATTTGAAAT
ATAATATATATTTTAAAAAAAAAATTTACAGTGTAGAATTTTTGGTAGTCCGGTTTGTTTTGAAATCAGTGGTGGTATTCAATATTTGA
TTAAATTTTGGTATGAATTTGTGTTGAAAAATAAAAAATAAGCGAGAAATTTGCGTGGCATATTATTTGTAATGTTCGAATATTCTCTC
GTACACCAAAAGGCTGCAG

## Claims

1. A mannosylglycerate synthase gene which results from a natural or artificial fusion between a complete or truncated mannosy-3-phosphoglycerate synthase gene and any complete or truncated phosphatase gene in the way that the resultant fusion protein is capable of formation of mannosylglycerate, with a nucleotide sequence shown, for example, in SEQ ID N°1.

2. A mannosylglycerate synthase protein as resulting from the expression of any of the genes or gene fusions of claim 1 with, for example, an amino acid sequence shown in SEQ ID N°2.

3. A recombinant plasmid that comprises any of the genes from claim 1 and which is recombined into any vector able to transform prokaryotic host cells as, for example, *Escherichia coli.*

4. A recombinant DNA molecule that comprises any of the genes as set forth in claim 1, simple or recombined into any vector for transformation of eukaryotic host cells, for example, *Saccharomyces cerevisiae*.

5. The utilization of organisms transformed with recombinant DNA according to claims 3 and 4 to produce any mannosylglycerate synthase protein resulting from claim 2.

6. The utilization of a mannosylglycerate synthase protein according to claim 2 to produce mannosylglycerate which comprises the preparation of cell extracts from the organisms of claim 5, for example *Escherichia coli,* and reacting the cell extracts or purified protein with precursors to produce mannosylglycerate.

7. The utilization of a transformed organism as depicted in claims 3 and 4, for example, *Saccharomyces cerevisiae,* for the production of mannosylglycerate.
